# EUROPEAN PATENT APPLICATION

(11) **EP 2 905 293 A1**
(43) Date of publication of application: **12.08.2015**
(21) Application number: 14162948.5
(22) Date of filing: 31.03.2014
(51) Int. Cl.: C08G 2/00, B01J 31/02, C07C 41/01, C07C 41/14, C07C 41/58, C07D 323/06, C07C 43/30, C07C 43/303, C07C 209/78, C07C 211/50, C08G 2/06, C08G 2/10, C08L 59/00

(54) **Process for catalytic synthesis of low-carbon polyether-based compound by using acidic ionic liquid**

(30) Priority: 09.12.2013 CN 201310659995
(71) Applicant: Lanzhou Institute Of Chemical Physics Chinese Academy of Sciences, Lanzhou, Gansu 730000 (CN); Suzhou OST Advanced Materials Co., Ltd., Suzhou Jiangsu 215123 (CN)
(72) Inventor: Xia, Chungu, 730000 Lanzhou (CN); Li, Zhen, 730000 Lanzhou (CN); Chen, Jing, 730000 Lanzhou (CN)
(74) Representative: Peters, Hajo

(57) **Abstract**

The present invention relates to a process for catalytic synthesis of a low-carbon polyether-based compound by using an acidic ionic liquid as the catalyst. The low-carbon polyether-based compound is prepared from an acetal compound and paraformaldehyde under the following conditions: reaction temperature of 70°C-200°C and reaction pressure of 0.2MPa-6MPa. The process is mainly characterized by avoiding the use of concentrated sulfuric acid or trifluoromethanesulfonic acid, which has strong corrosivity and will pollute the environment, as the catalyst. The ionic liquid being used has no corrosivity and will not pollute the environment, and has high selectivity for the low-carbon polyether-based compound product. Outstanding characteristics of the process include: the reaction system is free of water, the product can be very easily separated from the catalyst, the product can be widely applied, the ionic liquid can be used repeatedly, and therefore the outlook of the use of the process on industrial scale is very good.

## Description

### FIELD OF THE INVENTION

The present invention relates to a process for synthesis of a low-carbon polyether-based compound, more specifically, a process for catalytic synthesis of a low-carbon polyether-based compound from an acetal compound and paraformaldehyde by using an acidic ionic liquid as the catalyst.

### BACKGROUND OF THE INVENTION

A low-carbon polyether-based compound is a low molecular weight acetal polymer with an oxymethylene backbone, having a general formula of R(OCH₂)ₙOR, wherein n is an integer >1 and R can be C1-C10 alkyl. Such a compound has higher boiling point, high flash point, higher density, and low toxicity, and can be used in the electrolyte of fuel cells as a substitute for methanol (WO 2006/084993 A1, Journal of Power Sources, 2008,175:82). In the case that R is isobutyl, the compound is a solvent with high stability and high boiling point, and thus is of high value. Furthermore, such a polyether compound has high oxygen content in its molecule, and has high cetane number. Among others, the low-carbon polyether having a formula of CH₃(OCH₂)₃₋₈OCH₃, which is referred to as polyoxymethylene dimethyl ether, has similar chemical properties to diesel oil, is well intermiscible with diesel oil, and has higher stability. The researchers have found that the incorporation of 10%-20% into diesel oil can significantly decrease the condensation point of the diesel oil, improve the combustion properties of the diesel oil, improve the thermal efficiency, decrease the smoke number at most by 80%, decrease nitrogen oxides by 50%, and improve the overall performance of the diesel oil.

The raw materials for synthesizing the low-carbon polyether-based compound mainly comprise two parts, wherein the first part is a compound providing paraformaldehyde, including an aqueous solution of formaldehyde, trioxane, paraformaldehyde, etc; and the second part is a compound providing terminal alkyl, including low-carbon alcohols, dialkyl ethers, acetals, etc. There are various processes for synthesizing a low-carbon polyether-based compound. Concerning the raw materials, a low-carbon alcohol and formaldehyde are cheaper and easily available. However, formaldehyde conventionally presents in the form of an aqueous solution containing a large amount of water, bringing many disadvantages, e.g. decreasing the conversion per pass, bringing difficulty to the separation of the products, and increasing energy consumption. Furthermore, due to the use of an acidic catalyst in the reaction, the polyether product thus obtained might be hydrolyzed to hemiacetal, which is unstable and will decrease the flash point of the diesel oil mixture to some extent and in turn impair the quality of the diesel oil mixture. Hemiacetal, once formed, is hard to be separated from the product, since it has a similar boiling point to polyoxymethylene dimethyl ether.

Although a process for preparing formaldehyde by oxidation of dimethyl ether, which has been reported in US6392102, can decrease the content of water to some extent (concentration of formaldehyde >60%), the whole process, including reactive distillation and employing multiple heterogeneous reactors, distillation towers, absorption towers, and spraying towers, is so complicated that high costs of development and investment are needed. In addition, the cost of raw materials is relatively high when dimethyl ether is used as the substitute of methanol, in the preparation of formaldehyde.

EP1505049 describes, in an example, a process for synthesis of polyoxymethylene dimethyl ether from methanol and paraformoldehyde by using trifluoromethanesulfonic acid as the catalyst, wherein the selectivity for n=3-8 is 17.3%. Both trioxane and paraformaldehyde, as the substitutes for formaldehyde, are in solid forms and are safe in use and easy to be post-treated.

US2449269 describes a process for obtaining polyoxymethylene dimethyl ether having 2-4 formaldehyde units per molecule by heating methylal and paraformoldehyde or a concentrated solution of formaldehyde in the presence of sulfuric acid.

US 5746785 describes a process for obtaining polyoxymethylene dimethyl ether having molecular weight of 80-350 (corresponding to n=1-10), wherein a reaction is carried out within the temperature range of 150°C-240°C by using methylal and paraformoldehyde with a mole ratio of 1:5 or methanol and paraformoldehyde with a mole ratio of 1:3 as the raw materials.

EP 1070755 B1 (EP 1505049A1, US6534685) discloses a process for catalytic synthesis of polyoxymethylene dimethyl ether having 2-6 formaldehyde units per molecule from methylal and paraformoldehyde by using trifluoromethanesulfonic acid as the catalyst. The product prepared by such a process mainly consists of dimer. The selectivity for the product with n=2 is 49.6%, and the yield of the products with n=2-5 is 51.2%. The product is not suitable for diesel oil additives, since the dimer will decrease the flash point of the diesel oil.

Liquid acids with high corrosivity, such as sulfuric acid, are used in above mentioned references. Such liquid acids will significantly damage the equipments. And after the reaction is completed, they will remain in the same phase with the product, and therefore bring great difficulty to the separation of the products.

CN 101182367 (US 7560599) describes a process using methanol and trioxane as the raw materials and an ionic liquid as the catalyst. The amount of the ionic liquid catalyst occupies 0.01-10 wt% of the total reactants. The reaction temperature is 60°C-130°C and the reaction pressure is 0.5MPa-4MPa. The conversion of trioxane is up to 90.3% and the selectivity for the products with n=3-8 is up to 43.7%.

CN 101665414A discloses a process for catalytic synthesis of polyoxymethylene dimethyl ether from methylal and trioxane by using an ionic liquid as the catalyst. The conversion of trioxane is up to 95% and the selectivity for the products with n=3-8 is >43.7%, when the mole ratio of trioxane to methylal is 0.1-3.0, the amount of the catalyst occupies 0.5-8.0 wt% of the total reactants, the reaction temperature is 110°C-120°C, and the reaction pressure is 1.5MPa-3.0MPa. The ionic liquid catalyst has high catalytic efficiency, high selectivity, and low corrosion to equipments, and can be easily separated from the products.

The incorporation of trioxane, as the substitute for formaldehyde, can avoid the introduction of water into the reaction system. However, as a raw material, trioxane has higher cost than formaldehyde, methanol, etc. The incorporation of an acetal compound and paraformaldehyde, as the raw materials for the synthesis of low-carbon polyether-based compounds, is a satisfying route, since it can ensure that there is nearly no water exists or forms in the reaction system so as to avoid side reactions such as hydrolysis, etc., as well as efficiently reduce the production cost.

### SUMMARY OF THE INVENTION

An object of the present invention is to provide a process for the preparation of a low-carbon polyether-based compound by using an acetal compound and paraformaldehyde as the raw materials and an acidic ionic liquid as the catalyst, in order to overcome the disadvantages of prior liquid acid catalysts, e.g. corroding the equipments, bringing difficulty to the separation of the products, etc., and to avoid the adverse effects due to the formation of water in the reaction system. The process of the present invention has the following advantages: no water is produced in the reaction, and the catalyst has low corrosivity, can be readily separated from the products, and can be used repeatedly.

The reaction for the synthesis of the low-carbon polyether-based compound provided in the present invention is as follows:

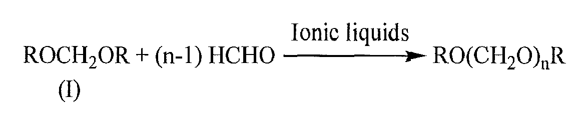

The compound of general formula (I) in the above reaction equation is a commercially available acetal compound, wherein R represents a linear or branched alkyl group having 1-5 carbon atoms.

The process according to the present invention, for catalytic synthesis of a low-carbon polyether-based compound by using an acidic ionic liquid as the catalyst, includes carrying out the reaction between an acetal compound and paraformaldehyde, in the presence of an acidic ionic liquid catalyst having a general formula of [M⁺][X⁻], so as to obtain the low-carbon polyether-based compound, wherein [M⁺] represents an organic cation having one or more sulphonyl groups, selected from an imidazolinium cation represented by the following formula 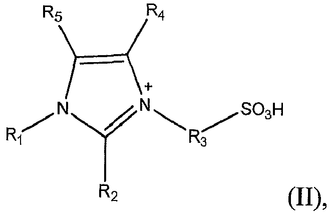 a pyridinium cation represented by the following formula 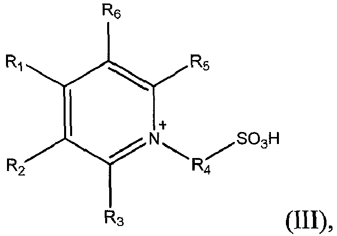 a quaternary ammonium cation represented by the following formula 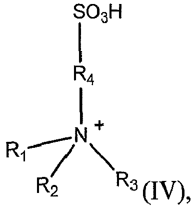 a quaternary phosphonium cation represented by the following formula 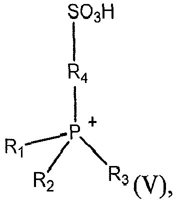 a pyrrolidinium cation represented by the following formula 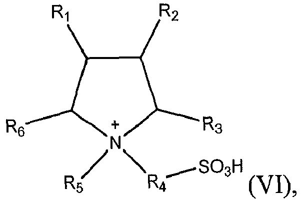 a guanidinium cation represented by the following formula 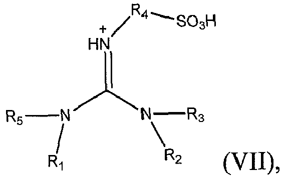 a morpholinium cation represented by the following formula 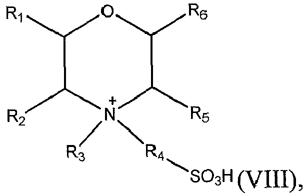 a dioxidothiomorpholinium cation represented by the following formula 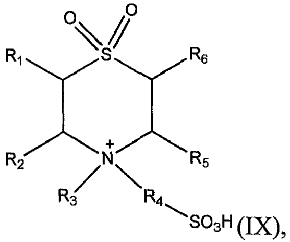 wherein
R₁, R₂, R₃, R₄, R₅, R₆, are identical to or different from one another, and independently, separately, or together, represents
H;
halogen;
alkyl (C1-C12), which can be partially or completely substituted by F, SO₃H, Cl or OH; and
[X⁻] is an anion selected from
Cl⁻, CₙH₂ₙ₊₁SO₃⁻, CF₃SO₃⁻, [N(SO₂CF₃)₂]⁻, [N(CF₃)₂]⁻, [C(CF₃SO₂)₃]⁻, BF₄⁻, PF₆⁻, CₙH₂ₙ₊₁CO₂⁻, CF₃CO₂⁻, CH₃(C₆H₄)SO₃⁻, EtSO₄⁻, MeSO₄⁻, SO₄²⁻, NO₃⁻, H₂PO₄⁻ or HSO₄⁻.

The low-carbon polyether-based compound according to the present invention has the general formula RO(CH₂O)ₙR, wherein R represents a linear or branched alkyl group having 1-5 carbon atoms and n is an integer >1.

The process according to the present invention is carried out under the following conditions: the amount of the catalyst occupies 1-10 wt% of the total reactants, the mole ratio of the acetal compound to the paraformaldehyde is 0.1-10:1, the reaction temperature is 70°C-200°C, and the reaction pressure is 0.2MPa -6MPa.

The mole ratio of the acetal compound to paraformaldehyde is preferably 0.1-5:1, the reaction temperature is preferably 80°C-150°C, and the reaction pressure is preferably 0.4MPa-4.0MPa. A two-phase system is produced after the reaction is completed, and the lower ionic liquid phase can be recycled and reused without any treatment after the upper organic phase is removed.

The key point of the synthesis process for the low-carbon polyether-based compound according to the present invention is the incorporation of an acid ionic liquid as the catalyst, the incorporation of an acetal compound and a paraformaldehyde as the raw materials, and the recovery of the ionic liquid by phase separation after the reaction has been completed. The advantages of the process are that
(1) by using an acidic ionic liquid as the catalyst, the corrosivity is low, the catalyst and the reaction products are separated into different phases so that the ionic liquid can be easily recycled, and the used ionic liquid can be reused for many times without any treatment;
(2) by using an acetal and paraformaldehyde as the raw material, the use of water-containing raw materials is avoided, the separation of the product is simple, and energy consumption is low;
(3) the products are well distributed and the use ratio of raw materials is high.

### DETAILED DESCRIPTION OF THE INVENTION

The ionic liquid according to the present invention can be prepared by the process provided in the following literature: J. Mol. Catal. A: Chem., 2005, 234:107; Synthesis, 2003, 17: 2626; J. Am. Chem. Soc., 2002, 124: 5962; Molecular Catalysis, 2008, 22(5): 392 and Chinese invention patent application ZL 200710305965.1; CN200910206549.5.

The following examples are provided only for illustrating the present invention.

### Example 1:

0.91g 3-butyl-1-(butane-4-sulfonic acid)imidazolinium bisulfate ionic liquid, 15.2g methylal and 3 g paraformaldehyde were sequentially added into a 100 ml reaction tank. The reaction pressure was brought to 2.0MPa by charging nitrogen. The reaction was heated to 130°C and stirred for 2h. The conversion of methylal was 48.1%, and the relative contents were: methylal 59.8%; n=2, 65.0%; n=3-8, 35.0%; and n>8, not detected, as determined by gas chromatography.

### Example 2:

Similar to Example 1, 1.34g 3-butyl-1-(butane-4-sulfonic acid)imidazolinium bisulfate ionic liquid, 21.7g ethylal and 5g paraformaldehyde were sequentially added. The reaction pressure was brought to 0.6MPa by charging nitrogen. The reaction was heated to 130°C and stirred for 2h. The conversion of ethylal was 45.3%, and the relative contents were: ethylal 38.4%; n=2, 53.6%; n=3-8, 46.3%; and n>8, 0.08%, as determined by gas chromatography.

### Example 3:

Similar to Example 1, 0.56g *N*-propanesulfonic acid pyridinium p-toluenesulfate ionic liquid, 11.4g methylal and 1.5g paraformaldehyde were sequentially added. The reaction pressure was brought to 3.0MPa by charging nitrogen. The reaction was heated to 130°C and stirred for 2h. The conversion of methylal was 27.9%, and the relative contents were: methylal 63.6%; n=2, 69.2%; n=3-8, 30.8%; and n>8, not detected, as determined by gas chromatography.

### Example 4:

Similar to Example 1, 1.0g 1,1,3,3-tetramethyl-2-(butane-4-sulfonic acid) guanidinium bisulfate ionic liquid, 15.2g methylal and 4.8g paraformaldehyde were sequentially added. The reaction pressure was brought to 0.6MPa by charging nitrogen. The reaction was heated to 130°C and stirred for 2h. The conversion of methylal was 43.9%, and the relative contents were: methylal 39.9%; n=2, 45.1%; n=3-8, 54.5%; and n>8, 0.36%, as determined by gas chromatography.

### Example 5:

Similar to Example 1, 0.72g 1,1,3,3-tetramethyl-2-(butane-4-sulfonic acid) guanidinium p-toluenesulfate ionic liquid, 11.4g methylal and 3.0g paraformaldehyde were sequentially added. The reaction pressure was brought to 0.6MPa by charging nitrogen. The reaction was heated to 140°C and stirred for 3h. The conversion of methylal was 45.9%, and the relative contents were: methylal 49.9%; n=2, 51.6%; n=3-8, 50.3%; and n>8, 0.1%, as determined by gas chromatography.

### Example 6:

Similar to Example 1, 1.92g 3-butyl-1-(butane-4-sulfonic acid)imidazolinium bisulfate ionic liquid, 33.3g ethylal and 5g paraformaldehyde were sequentially added. The reaction pressure was brought to 0.6MPa by charging nitrogen. The reaction was heated to 130°C and stirred for 2h. The conversion of ethylal was 37.2%, and the relative contents were: ethylal, 57.3%; n=2, 61.2%; n=3-8, 35.7%; and n>8, 0.2%, as determined by gas chromatography.

### Example 7:

Similar to Example 1, 0.70g triethyl(propane-3-sulfonic acid)ammonium trifluoromethanesulfate ionic liquid, 15.2g methylal and 2.4g paraformaldehyde were sequentially added. The reaction pressure was brought to 0.6MPa by charging nitrogen. The reaction was heated to 120°C and stirred for 2h. The conversion of methylal was 30.3%, and the relative contents were: methylal 58.9%; n=2, 63.4%; n=3-8, 36.6%; and n>8, not detected, as determined by gas chromatography.

### Example 8:

Similar to Example 1, 1.27g tributyl(propane-3-sulfonic acid)phosphonium bisulfate ionic liquid, 18.2g methylal and 7.2g paraformaldehyde were sequentially added. The reaction pressure was brought to 2.5MPa by charging nitrogen. The reaction was heated to 130°C and stirred for 2h. The conversion of methylal was 21.0%, and the relative contents were: methylal, 50.2%; n=2, 45.3%; n=3-8, 54.7%; and n>8, not detected, as determined by gas chromatography.

### Example 9:

Similar to Example 1, 1.06g *N*-butyl-*N*-(butane-4-sulfonic acid)pyrrolidinium bisulfate ionic liquid, 15.2g methylal and 6.0g paraformaldehyde were sequentially added. The reaction pressure was brought to 2.5MPa by charging nitrogen. The reaction was heated to 130°C and stirred for 2h. The conversion of methylal was 48.1%, and the relative contents were: methylal, 35.1%; n=2, 39.8%; n=3-8, 59.3%; and n>8, 0.88%, as determined by gas chromatography.

### Example 10:

Similar to Example 1, 1.0g *N*-methyl-*N*-(butane-4-sulfonic acid)dioxidothiomorpholinium bisulfate ionic liquid, 15.2g methylal and 4.8g paraformaldehyde were sequentially added. The reaction pressure was brought to 3.0MPa by charging nitrogen. The reaction was heated to 130°C and stirred for 2h. The conversion of methylal was 40.7%, and the relative contents were: methylal, 50.8%; n=2, 59.0%; n=3-8, 41.0%; and n>8, not detected, as determined by gas chromatography.

## Claims

1. A process for catalytic synthesis of a low-carbon polyether-based compound by using an acidic ionic liquid, **characterized in that** the low-carbon polyether-based compound are obtained by the reaction between an acetal compound and paraformaldehyde, in the presence of an acidic ionic liquid catalyst having a general formula of [M⁺][X⁻], wherein
[M⁺] represents an organic cation having one or more sulphonyl groups, selected from an imidazolinium cation represented by the following formula 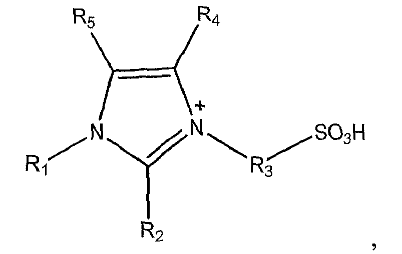 a pyridinium cation represented by the following formula 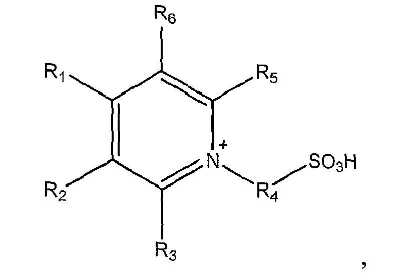 a quaternary ammonium cation represented by the following formula 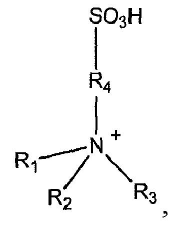 a quaternary phosphonium cation represented by the following formula 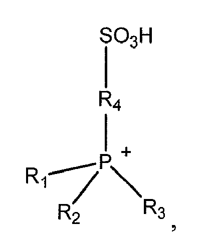 a pyrrolidinium cation represented by the following formula 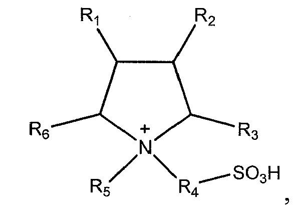 a guanidinium cation represented by the following formula 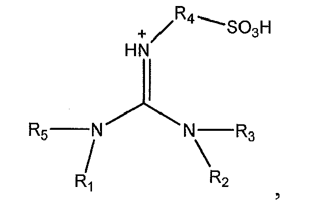 a morpholinium cation represented by the following formula 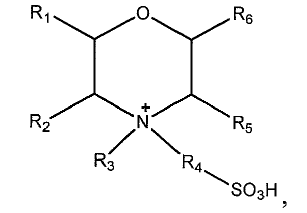 a dioxidothiomorpholinium cation represented by the following formula 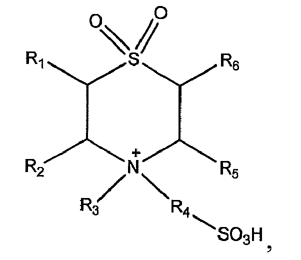 wherein,
R₁, R₂, R₃, R₄, R₅, R₆, are identical to or different from one another, and independently, separately, or together, represents
H;
halogen;
alkyl(C1-C12), which can be partially or completely substituted by F, SO₃H, Cl or OH;
and
[X⁻] is an anion selected from
Cl⁻, CₙH₂ₙ₊₁SO₃⁻, CF₃SO₃⁻, [N(SO₂CF₃)₂]⁻, [N(CF₃)₂]⁻, [C(CF₃SO₂)₃]⁻, BF₄⁻, PF₆⁻, CₙH₂ₙ₊₁CO₂⁻, CF₃CO₂⁻, CH₃(C₆H₄)SO₃⁻, EtSO₄⁻, MeSO₄⁻, SO₄²⁻, NO₃⁻, H₂PO₄⁻ or HSO₄⁻.

2. The process according to claim 1, **characterized in that** the acetal compound is a compound having following general formula (I)
ROCH₂OR (I),
wherein R represents a linear or branched alkyl group having 1-5 carbon atoms.

3. The process according to claim 1, **characterized in that** the low-carbon polyether-based compound has the general formula RO(CH₂O)ₙR, wherein R represents a linear or branched alkyl group having 1-5 carbon atoms and n is an integer >1.

4. The process according to claim 1, **characterized in that** the amount of the acidic ionic liquid catalyst occupies 1-10 wt% of the total reactants.

5. The process according to claim 1, **characterized in that** the mole ratio of the acetal compound to paraformaldehyde is 0.1-5:1, the reaction temperature is 80°C-150°C, and the reaction pressure is 0.4MPa-4.0MPa.

6. The process according to any one of claims 1-5, **characterized in that** a two-phase system is produced after the reaction is completed, and the lower ionic liquid phase can be recycled and reused without any treatment after the upper organic phase is removed.
